# EUROPEAN PATENT SPECIFICATION

(11) **EP 1 328 657 B1**
(45) Date of publication and mention of the grant of the patent: **01.03.2006**
(21) Application number: 01980238.8
(22) Date of filing: 08.08.2001
(51) Int. Cl.: C12Q 1/48, G01N 33/68

(54) **PROCESS FOR DETECTING SERINE/THREONINE KINASE ACTIVITY**
VERFAHREN ZUM NACHWEIS VON SERINE/THREONINE KINASE AKTIVITÄT
PROCEDE DE DETECTION DE L'ACTIVITE DE LA KINASE THREONINE/SERINE

(30) Priority: 11.08.2000 EP 00117457
(43) Date of publication of application: 23.07.2003
(73) Proprietor: Evotec AG, 22525 Hamburg (DE); Pfizer Limited, Sandwich, Kent CT13 9NJ (GB)
(72) Inventor: KRÄMER, Joachim, Evotec AG, 22525 Hamburg (DE); MANDER, Thomas, Evotec AG, 22525 Hamburg (DE); BETHELL, Richard,, Montreal Quebec H3G 2P5 (CA); BENSON, Neil, c/o Pfizer Limited, Kent CT13 9NJ (GB); BOYD, Helen, c/o Pfizer Limited, Kent CT13 9NJ (GB); GREENGRASS, Pam,, Kent CT13 9PL (GB); KINLOCH, Ross, c/o Pfizer Limited, Kent CT13 9NJ (GB)
(74) Representative: Hertz, Oliver
(86) International application number: PCT/EP2001/009186
(87) International publication number: WO 2002/014542

(56) References cited:
- WO-A-99/29894
- KHOKHLATCHEV ANDREI ET AL: "Reconstitution of mitogen-activated protein kinase phosphorylation cascades in bacteria: Efficient synthesis of active protein kinase." JOURNAL OF BIOLOGICAL CHEMISTRY, vol. 272, no. 17, 1997, pages 11057-11062, XP002163437 ISSN: 0021-9258
- MARTIN HUMBERTO ET AL: "Regulatory mechanisms for modulation of signaling through the cell integrity Slt2-mediated pathway in Saccharomyces cerevisiae." JOURNAL OF BIOLOGICAL CHEMISTRY, vol. 275, no. 2, 14 January 2000 (2000-01-14), pages 1511-1519, XP002225502 ISSN: 0021-9258
- SEETHALA RAMAKRISHNA ET AL: "A fluorescence polarization competition immunoassay for tyrosine kinases" ANALYTICAL BIOCHEMISTRY,ACADEMIC PRESS, SAN DIEGO, CA,US, vol. 255, 15 January 1998 (1998-01-15), pages 257-262, XP002150326 ISSN: 0003-2697 cited in the application
- WU JINZI J ET AL: "Identification of a high-affinity anti-phosphoserine antibody for the development of a homogeneous fluorescence polarization assay of protein kinase C." JOURNAL OF BIOMOLECULAR SCREENING, vol. 5, no. 1, February 2000 (2000-02), pages 23-30, XP000992190 ISSN: 1087-0571
- LAWLER SEAN ET AL: "Synergistic activation of SAPK1/JNK1 by two MAP kinase kinases in vitro." CURRENT BIOLOGY, vol. 8, no. 25, 17 December 1998 (1998-12-17), pages 1387-1390, XP000991273 ISSN: 0960-9822 cited in the application
- HOFFMANN RALF ET AL: "Phosphorylation of the C-terminal sites of human p53 reduces non-sequence-specific DNA binding as modeled with synthetic peptides." BIOCHEMISTRY, vol. 37, no. 39, pages 13755-13764, XP002225711 ISSN: 0006-2960
- DATABASE BIOSIS [Online] BIOSCIENCES INFORMATION SERVICE, PHILADELPHIA, PA, US; August 1999 (1999-08) BARANCIK MIROSLAV ET AL: "Okadaic acid and anisomycin are protective and stimulate the SAPK/JNK pathway." Database accession no. PREV199900492152 XP002225692 & JOURNAL OF CARDIOVASCULAR PHARMACOLOGY, vol. 34, no. 2, August 1999 (1999-08), pages 182-190, ISSN: 0160-2446
- FLEMING YVONNE ET AL: "Synergistic activation of stress-activated protein kinase 1/c-Jun N-terminal kinase (SAPK1/JNK) isoforms by mitogen-activated protein kinase kinase 4 (MKK4) and MKK7." BIOCHEMICAL JOURNAL, vol. 352, no. 1, 15 November 2000 (2000-11-15), pages 145-154, XP000990277 ISSN: 0264-6021 cited in the application

## Description

The present invention relates to a process for detecting threonine or serine (threonine/serine) kinase activity in an immunoassay. The invention further relates to a kit for carrying out the assay and to a preferably luminescently labelled ligand.

Protein kinases usually catalyse the transfer of the γ-phosphate group from ATP to a serine, threonine or tyrosine residue of an acceptor protein. These enzymes play an important role in signal transduction within cells. Detecting the activity of protein kinases would be useful for the high-throughput screening of chemical libraries. Inhibitors or activators of kinase activity, particularly low-molecular weight compounds, could eventually be developed into drugs used for the treatment of e.g. ischaemic heart disease, liver failure, diabetic neuropathies, stroke, neurodegenerative disorders including Parkinsons disease and Alzheimers disease, inflammatory diseases including asthma, rheumatoid arthritis, inflammatory bowel disease, septic shock and cancer.

It is known that mitogen-activated protein kinases (MAPK) (also referred to as stress activated protein kinases, SAPK) mediate many of the cellular effects of growth factors, cytokines and stress, leading to cell growth, differentiation and oncogenesis. MAPK/SAPK activation requires dual phosphorylation on threonine and tyrosine within the motif threonine-Xaa-tyrosine, where Xaa represents proline in the c-Jun NH2-terminal kinases (JNKs). This activation is catalysed by several MAPK-kinases (e.g. MKK4(SKK1) or MKK7 (SKK4)) which phosphorylate both the threonine and tyrosine residues of the threonine(183)-proline-tyrosine(185) motif within the active site loop of JNK1/2/3. The MKK7 kinase has a high preference for the threonine residue whereas MKK4 preferentially phosphorylates tyrosine. This synergistic activation of JNK1/2/3 is described by Lawler S. et al in Current Biology 8, 1387 to 1390 (1998) and Fleming Y et al. (Biochemical Journal, 352, 145 - 154, 2000). Up to now the identification of an appropriate substrate which could be used to screen for specific MKK7 inhibitors is complicated by the fact that there are no synthetic substrate peptides described in the literature. The situation becomes even more critical in homogeneous assays as a potential MKK7 substrate peptide should have both a reasonable Km for MKK7 and a high affinity for the phospho-threonine specific detection antibody in parallel. Although generic anti-phospho-tyrosine antibodies with high affinity and specificity are available for tyrosine-directed kinases (e.g. p60c-src kinase), attempts to develop generic anti-phospho-threonine/anti-phospho-serine antibodies for the same purpose have been to date less successful.

Antibodies which bind to phosphorylated threonine or serine residues with high affinity only recognize the phosphorylated amino acid in the context of the surrounding peptide sequence. For antibodies binding to JNK1/2/3 or peptide derivatives thereof, recognition is therefore dependent on two criteria: 1) MKK7-dependent phosphorylation of threonine and 2) phosphorylation of the tyrosine amino acid residue. If one of the two criteria is not fulfilled, an antibody raised against fully activated JNK1/2/3 will not specifically detect the phosphorylation site.

In *Anal. Biochem.* 255, 257 to 262 (1998) a fluorescence polarization (FP) competition immunoassay for tyrosine kinases using an appropriate substrate for this kinase is described. The kinase reaction was performed by incubation of the peptide substrate with ATP and lymphoid T-cell protein tyrosine kinase followed by termination of the reaction with EDTA plus a fluorescein-phosphopeptide. Following the addition of an anti-phosphotyrosine antibody, the fluorescence polarization signal was measured. The phosphorylated product formed in the assay competes with the fluorescein-phosphopeptide for binding to the anti-phosphotyrosine antibody. The kinase activity results in a reduction of the FP signal and the FP signal is therefore inversely proportional to the phosphorylated product formed in the reaction.

However, such an approach for a generic assay principle for serine/threonine kinases is not realizable to date. This is because there are no anti-phospho-serine/-threonine antibodies available that bind specifically, and with high affinity, to phosphoserine or phosphothreonine residues in the absence of additional specific amino acid sequences adjacent to the phosphorylated amino acid.

Khokhlatchev et al. (J. Biol. Chem., Vol. 272, No. 17, (1997), pages 11057-11062) describe an in vivo method for following MEK kinase activity in which MEKK is coexpressed with either extracellular signal-regulated kinase 2 (ERK2) or a stress activated protein kinase (SAPK). In the normal course of events MEKK phosphorylates MEK which in turn phosphorylates ERK2 or SAPK2. This method, however, does not provide an in vitro immunoassay for detecting serine/threonine kinase activity wherein the use of specific high affinity anti-phospho-serine/-threonine antibodies is not a prerequisite.

It was therefore the object of the present invention to establish an assay method for detecting serine/threonine kinases or their enzymatic activity. The assay method is highly reliable and simple to perform without the need to develop specific high affinity anti-phospho-serine/-threonine antibodies.

This object has been solved by the assay process according to the features of claim 1.

As used herein, the term "kinase" refers to an enzyme capable of phosphorylating its substrate. A "serine/threonine kinase" refers to an enzyme capable of phosphorylating its substrate at a serine or threonine residue. The term "bis-phosphorylated" or "double phosphorylated" indicates that a protein or peptide comprising the sequence motif -Z-X-Y- or -Y-X-Z- is phosphorylated both at the Z and the Y position. Unless otherwise indicated, the term "bis-phosphorylated" or "double phosphorylated" as used herein does not exclude the possibility that said protein or peptide is further phosphorylated at positions other than Y and Z.

The present invention relates to a process for detecting threonine or serine kinase activity in an immunoassay which comprises the following steps:
a) providing a protein or peptide comprising the sequence motif

   -Z-X-Y - or -Y-X-Z-

   wherein
   Z = threonine or serine
   X = a sequence of preferably between 1 and 1000 amino acids which may be the same or different
   Y = tyrosine, threonine or serine
   as a substrate for threonine or serine kinases, said protein or peptide being pre-phosphorylated at the Y position;
b) incubating the peptide or protein with a phosphate donor and a threonine or serine kinase to form a bis-phosphorylated protein or peptide;
c) adding an antibody having a specificity to the peptide/protein that has been phosphorylated at threonine or serine in the Z position; and
d) detecting the threonine or serine kinase activity.

Or to put it in other words, the invention relates to a method for determining the phosphorylating activity of an enzyme which comprises the steps of:
a) combining said enzyme with
   - a protein or peptide comprising the sequence motif

      -Z-X-Y- or -Y-X-Z-

      wherein
      Z = threonine or serine
      X = a sequence of preferably between 1 and 1000 amino acids which may be the same or different
      Y = phosphotyrosine, phosphothreonine or phosphoserine,
      said protein or peptide capable of being phosphorylated at the Z position by said enzyme;
   - a phosphate donor; and
   - an antibody having a specificity to a peptide/protein which is phosphorylated both at the Y and Z position; and
b) detecting the enzyme activity.
   All assay components might be combined simultaneously or stepwise. The substrate might in particular be combined with said enzyme before the addition of said antibody.

Particularly, the antibody has a specificity to the bis-phosphorylated product of the kinase reaction. By detecting the presence, absence or amount of the product-antibody complex, a threonine or serine kinase activity can be measured. Or to put it in another way: the invention also allows for the determination of the phosphorylation of a substrate molecule by an enzyme at an amino acid selected from the group consisting of serine and threonine.

The present invention also relates to a kit for detecting threonine or serine kinase activity in an immunoassay which comprises the following components:
- preferably a threonine or serine kinase;
- a pre-phosphorylated substrate as defined above;
- an antibody as defined above; and
- preferably reaction buffers including a phosphate donor.

The present invention also relates to a labelled, preferably luminescently labelled, ligand for use in a serine/threonine kinase assay comprising the sequence motif

-Z-X-Y- or -Y-X-Z-

wherein
Z = threonine or serine
X = a sequence of preferably between 1 and 1000 amino acids which may be the same or different .
Y = tyrosine, threonine or serine
said protein or peptide ligand being phosphorylated at the Z and Y position.

The subclaims define preferred embodiments of the process of the present invention.

The process of the present invention as well as the kit and the labelled ligand may be used for screening for specific modulators of serine or threonine kinase activity. They are particularly suitable for screening compound libraries in order to locate molecules which inhibit or activate kinases. These molecules may be promising candidates for designing drugs used for the treatment of e.g. ischaemic heart disease, liver failure, diabetic neuropathies, stroke, neurodegenerative disorders including Parkinsons' disease and Alzheimers' disease, inflammatory diseases including asthma, rheumatoid arthritis, inflammatory bowel disease, septic shock and cancer. Screening for an agent capable of increasing or decreasing the phosphorylating activity of the enzyme typically comprises the steps of (i) performing the process as outlined above in the presence and in the absence of said agent; and (ii) comparing the activity of said enzyme in the presence of said agent with the activity of said enzyme in the absence of said agent to determine whether the phosphorylating activity of said enzyme in the presence of said agent is increased or decreased. However, the subject of the present invention might also be useful for the study of the functionality of an enzyme. For instance, one could check whether an unknown protein belongs to the group of serine/threonine kinases or whether a specific serine/threonine kinase is biologically active.

The accompanying figures illustrate the present invention. Abbreviations are used as follows:
- P1°: H-Lys-Phe-Met-Met-Thr-Pro-pTyr-Val-Val-Thr-Arg-NH2
- P1*: H-Lys-Phe-Met-Met-pThr-Pro-Tyr-Val-Val-Thr-Arg-NH2
- P1*°: H-Lys-Phe-Met-Met-pThr-Pro-pTyr-Val-Val-Thr-Arg-NH2
- TAMRA-P1*°: 
5-TAMRA-AEEA-Lys-Phe-Met-Met-pThr-Pro-pTyr-Val-Val-Thr-Arg-
NH2
- TAMRA: 5'-(6-carboxytetramethylrhodamine)
- AEEA: 8-amino-3,6-dioxaoctanoic acid linker

In the figures the following is shown:
- Figure 1: is a schematic drawing of a preferred embodiment of the assay principle of the present invention.
- Figure 2: is a schematic diagram showing the binding of the polyclonal JNK antibody to TAMRA-labelled P1*°-peptide; as a control, no binding is measured for TAMRA-labelled P1°-peptide.
- Figure 3: is a schematic diagram showing the competition of the binding of the polyclonal JNK antibody to the TAMRA-labelled P1*°-peptide using mono-phosphorylated P1°-and P1*-peptides and double-phosphorylated P1*°-peptide (determination of the IC50 for the P1*°-peptide competitor).
- Figure 4: is a schematic diagram showing the competition of the binding of the polyclonal JNK antibody to the TAMRA-labelled P1*°-peptide in presence of 100 µM P1°-peptide (substrate) using P1*°-peptide (determination of the IC50 for the P1*°-peptide competitor under MKK7 kinase assay conditions).
- Figure 5: is a schematic diagram showing the phosphorylation of P1°-peptide substrate by MKK7 kinase at different P1°-peptide concentrations (determination of the Km for P1°-peptide).
- Figure 6: is a schematic diagram showing the phosphorylation of P1°-peptide substrate by MKK7 kinase at different ATP concentrations (determination of the Km for ATP).
- Figure 7: is a schematic diagram showing the inhibition of the MKK7 kinase-dependent phosphorylation of P1°-peptide substrate by Staurosporine (determination of the IC50 for Staurosporine).

According to the invention, a protein or peptide comprising the sequence motif -Z-X-Y- or -Y-X-Z-, wherein Z = threonine or serine; X = a sequence of preferably between 1 and 1000 amino acids which may be the same or different; Y = tyrosine, threonine or serine, is used as a substrate for the threonine or serine kinase. According to the present invention said protein or peptide is pre-phosphorylated at the Y position. It has been shown that the proteins and peptides being pre-phosphorylated at the Y residue can be successfully used as synthetic substrates for threonine or serine kinases.

In the sequence motif, any amino acid or sequence of amino acids (X) can be inserted between Z and Y. The number of amino acids is preferably in the range of 1 to 1000 amino acids. A range of 1 to 1000 is preferred to include both linear and conformational antibody epitopes. X is particularly at least one amino acid, any other short amino acid sequences having at least two amino acids, such as oligopeptides, being also included. Preferably, X is proline or glutamate or glycine.

The antibody used is usually a monoclonal or polyclonal antibody.

It has been shown that a particularly preferred antibody is a polyclonal antibody specific for bis-phosphorylated, in particular activated JNK. Such antibodies are commercially available.

It has been revealed that the antibody specifically recognizes a phosphorylated threonine or serine residue at the Z position within both a synthetic substrate peptide or bis-phosphorylated, in particular activated JNK.

The immunoassay according to the present invention is amenable to be carried out in a homogeneous assay format (i.e. "mix, incubate, and read"). This assay format is very advantageous because it is suitable for both high throughput screening (HTS) of potential drugs and secondary assays.

The immunoassay of the present invention may be performed as a direct binding immunoassay, preferably a homogeneous direct binding immunoassay.

In the direct binding immunoassay, a labelled peptide/protein or a labelled antibody is used. The labelling may be carried out according to conventional standard techniques. Preferably, the peptide/protein or antibody is labelled using a luminescent or a radioactive tag or by using specific labelling molecules such as a reporter enzyme or an affinity ligand.

The assay of the present invention may also be performed as a competition immunoassay, preferably a homogeneous indirect binding immunoassay.

In this indirect binding immunoassay, a labelled double-phosphorylated ligand is added to compete with the double-phosphorylated peptide or protein for binding to the antibody. The ligand is preferably labelled using a luminescent or a radioactive tag or by using specific labelling molecules such as a reporter enzyme or an affinity ligand.

In general, fluorescence detection offers a preferred alternative to the use of radiotracers, as fluorescence not only offers detection limits comparable to those of radioactivity but also eliminates the cost of radioactive waste disposal.

In case of using a protein as a substrate containing the above motif, the JNK protein is preferably used, which is the c-Jun N-terminal kinase. JNK kinase is also known in the literature as the stress-activated protein kinase 1 (SAPK1).

When it is more convenient to use a peptide substrate, the peptide sequence is preferably selected from the active-site loop, e.g. for MKK7 from the JNK1/2/3 active site. For instance, said peptide for MKK7 comprises or is composed of the amino acid sequence H-Lys-Phe-Met-Met-Thr-Pro-pTyr-Val-Val-Thr-Arg-NH₂, wherein p means phosphorylated.

When the incubation of the protein or peptide is carried out in the presence of a threonine kinase, the threonine kinase is preferably a mitogen-activated protein kinase kinase(MKK), alternatively called stress activated protein kinase (SKK). More particularly, the kinase is MKK7/SKK4.

According to one embodiment of the process of the present invention, an antibody is added preferably at the start of the reaction having a specificity to the bis-phosphorylated sequence, preferably to phosphorylated threonine or serine. During the enzyme reaction any double phosphorylated product formed is bound by the antibody. Then, at the end of the enzyme reaction, a labelled, double phosphorylated ligand is added, either with or without the concomitant addition of a reagent to stop the reaction, to compete with the double phosphorylated protein or peptide for binding to the antibody. This indirect assay principle is based on the fact that if the substrate becomes double phosphorylated by the kinase such as MKK7, it will compete with the labelled, double phosphorylated peptide-antibody complex. This strategy has the advantage of using non-labelled substrates and a fixed antigen-antibody complex that gives the read-out.

Usually, the double phosphorylated ligand is labelled by common techniques, using a luminescent or a radioactive tag or by molecules such as a reporter enzyme or an affinity ligand. Preferably, the ligand is labelled by a fluorescent dye. Particularly, the ligand comprises or is composed of the sequence 5-TAMRA-AEEA-Lys-Phe-Met-Met-pThr-Pro-pTyr-Val-Val-Thr-Arg-NH₂.

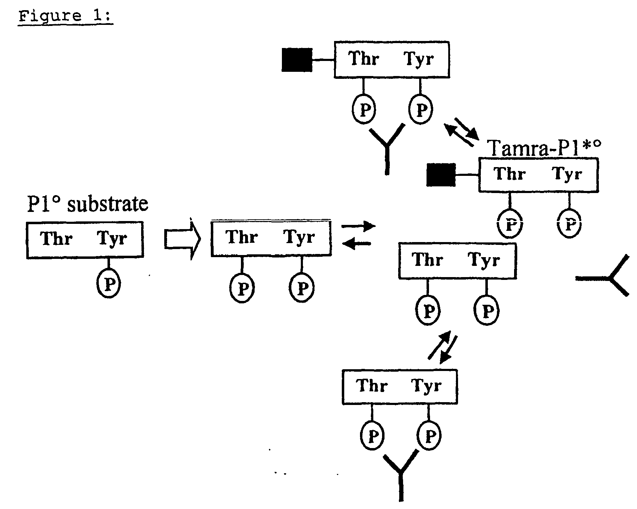

In Fig. 1 the indirect assay principle is illustratively shown. The P1° substrate having the amino acid sequence H-Lys-Phe-Met-Met-Thr-Pro-pTyr-Val-Val-Thr-Arg-NH₂ (p means phosporylated) becomes phosphorylated at the threonine residue in the presence of ATP and the MKK7 kinase. This double-phosphorylated peptide (ligand) competes with the labelled TAMRA-P1*° peptide having the sequence 5-TAMRA-AEEA-Lys-Phe-Met-Met-pThr-Pro-pTyr-Val-Val-Thr-Arg-NH₂ for binding to the antibody.

In a preferred embodiment, the assay may be conveniently performed as a fluorescence immunoassay, in particular fluorescence polarization (FP) immunoassay. However, other fluorescence techniques such as fluorescence correlation spectroscopy (FCS), fluorescence intensity distribution analysis (FIDA), fluorescence quenching (FQ) or fluorescence resonance energy transfer (FRET) might be applied.

According to the invention a kit for detecting threonine or serine kinase activity is provided. The kit comprises preferably the following components:
1. a threonine or serine kinase such as natural or recombinant enzyme;
2. a substrate as outlined above, e.g. in form of a monophosphorylated-peptide or mono-phosphorylated protein;
3. an antibody as outlined above;
4. reaction buffers including e.g. either MnCl₂ or MgCl₂, and a phosphate donor such as ATP;
5. reaction vials; and
6. protocol

It is preferred that the kit includes a substrate and an antibody as defined above and optionally also reaction buffers, vials and protocols. The user of the kit may have the option to choose an appropriate kinase himself. In a further embodiment, the kit might also include a specific threonine or serine kinase.

The kit preferably further comprises a labelled (in particular luminescently labelled) ligand, which ligand comprises a sequence motif

-Z-X-Y- or -Y-X-Z-

wherein Z = threonine or serine
X = a sequence of preferably between 1 and 1000 amino acids which may be the same or different
Y = tyrosine, threonine or serine
This ligand acts as a competitor for performing the assay according to the present invention, said competitor being pre-phosphorylated at the Z and Y positions.

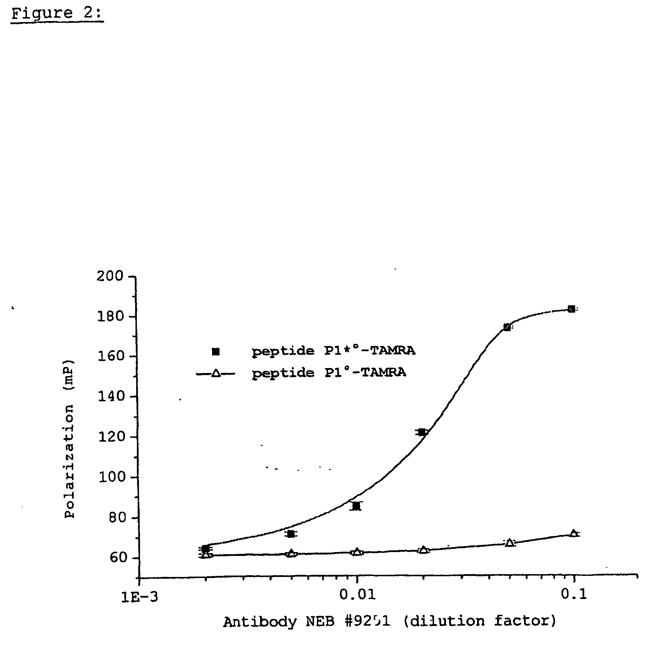

It has been revealed that the commercially available polyclonal anti-phospho-kinase antibodies are particularly useful in the assay of the present invention. As can be seen in Fig. 2 (ref. Example 1), the polyclonal phospho-JNK antibody detects all three isoforms of the JNK proteins only when activated by dual phosphorylation at threonine₁₈₃ and tyrosine₁₈₅. The polarization values dramatically increase when using the double-phosphorylated peptide substrate P1*° in contrast to mono-phosphorylated labelled peptide P1° (sequence see above).

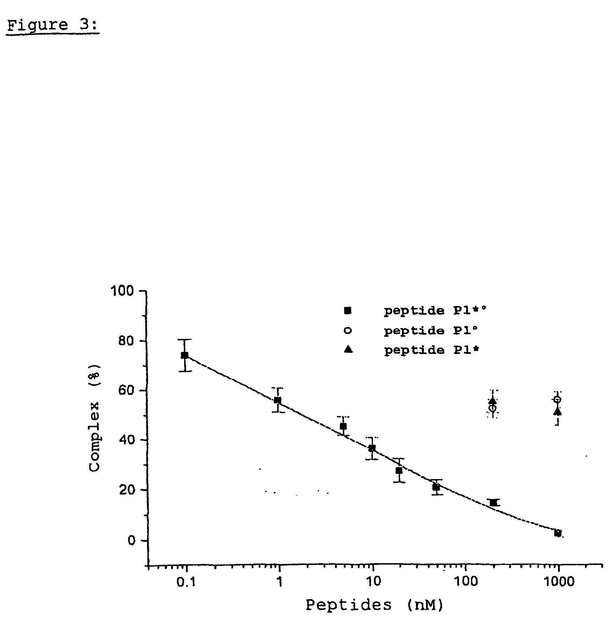

In Fig. 3 (ref. Example 2) the binding of the same polyclonal antibody to TAMRA-labelled peptide P1*°(5 nM) in competition with the peptides P1° (mono-phosphorylated, sequence see above), peptide P1* (mono-phosphorylated having the sequence H-Lys-Phe-Met-Met-pThr-Pro-Tyr-Val-Val-Thr-Arg-NH₂) or peptide P1*° (double phosphorylated, sequence see above) is presented. As can be deduced from the complex formation, only bis-phosphorylated peptide P1*° is able to compete effectively with the TAMRA-labelled peptide P1*° for binding to the antibody while the other mono-phosphorylated peptides displace less than 50% of the bound ligand even at high concentrations (up to 1 µM).

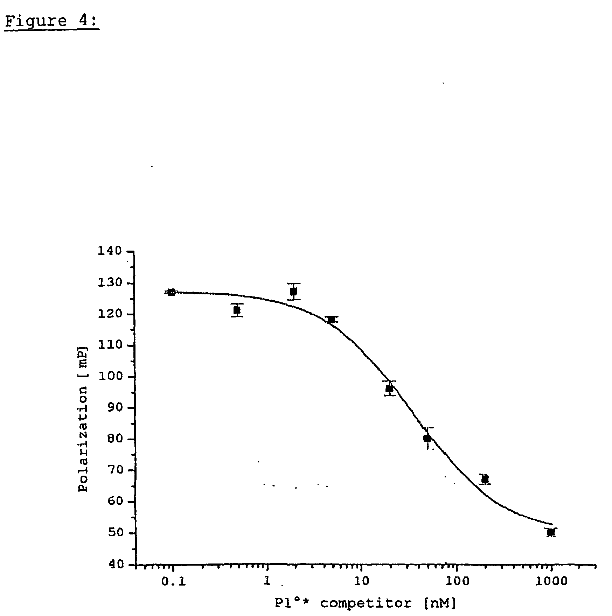

In Fig. 4 (ref. Example 3) the binding of the same polyclonal antibody to TAMRA-labelled peptide P1*° (5 nM) is displaced by bis-phosphorylated competitor peptide in presence of P1° peptide substrate (100 µM). These conditions reflect the concentration at which P1° peptide is used as a substrate for the MKK7 kinase. As can be seen from the result, the presence of mono-phosphorylated P1° peptide does not reduce the dynamic range of the described kinase assay.

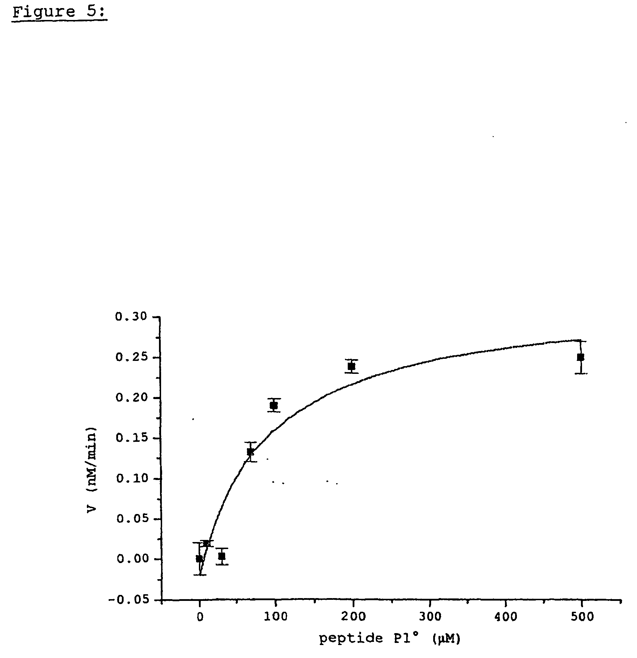

In Fig. 5 (ref. Example 4) the rate of phosphorylation of P1° peptide substrate by MKK7 kinase is determined at various P1° peptide concentrations. Efficient phosphorylation of P1° peptide is measured. The formed double-phosphorylated P1*° peptide product can be detected as it competes with the TAMRA-labelled P1*° peptide for the binding to the same polyclonal phospho-JNK antibody. As a results, a drop of the fluorescence polarization is detected which is inversely proportional to MKK7 kinase activity. From the detected signals, the Michaelis-Menten constant (Km), reflecting the half-maximal phosphorylation rate, was determined for P1° peptide.

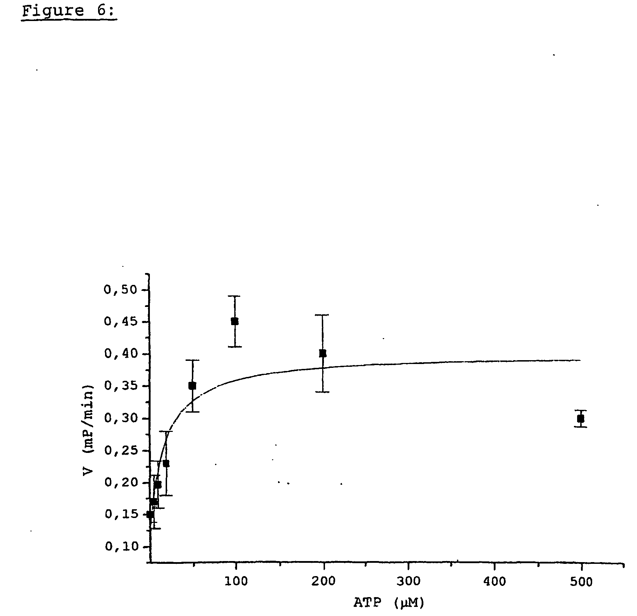

In Fig. 6 (ref. Example 5) the rate of phosphorylation of P1° peptide substrate by MKK7 kinase is determined at different ATP concentrations. Efficient phosphorylation of P1° peptide is dependent on the optimal ATP concentration. The formation of the bis-phosphorylated P1*° peptide product is detected using the same competition assay as described in Fig 5. From the detected fluorescence polarization signals, the Michaelis-Menten constant (Km), reflecting the half-maximal phosphorylation rate, was determined for ATP.

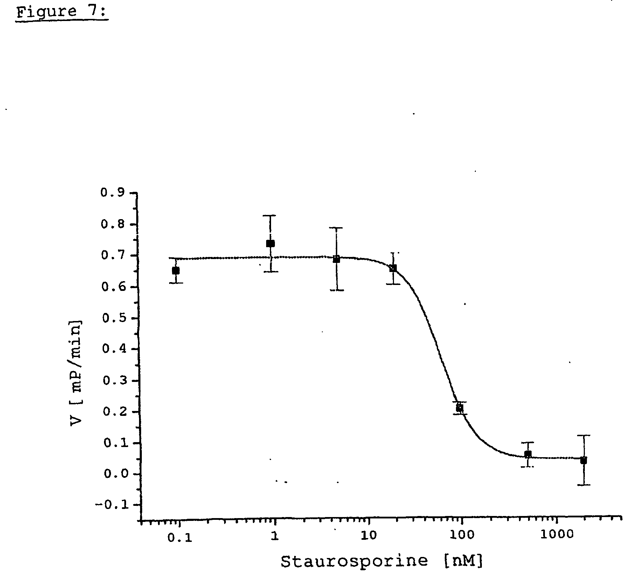

In Fig. 7 (ref. Example 6) the inhibition of the phosphorylation of P1° peptide substrate by MKK7 kinase is determined for the kinase inhibitor staurosporine. The inhibition of MKK7 kinase is detected using the same competition assay as described in Fig 5. As a result, no competition of the TAMRA-labelled P1*° peptide from the same polyclonal phospho-JNK antibody is detected at high Staurosporine concentrations. The half-maximal inhibitory concentration (IC50) was calculated for Staurosporine.

The following examples illustrate the assay of the present invention. The experiments described hereinafter show the feasibility to monitor the phosphorylating activity of serine/threonine kinases in a homogeneous assay format amenable to high throughput screening. The experiments are based on the finding that a modification - phosphorylation at the Y position - of the substrate in spacial proximity to the Z-site of phosphorylation not only enhances the affinity of the phosphorylated product to the detection antibody but also results in a high specificity for the phosphorylated Z position. Besides having the advantage of affinity enhancement of the antibody, the modification of the substrate according to the present invention turns out to have an unexpected positive effect on the kinetics of the kinase reaction as well.

### Example 1:

### Measurement of the binding affinity (KD) of antibody NEB #9251:

This polyclonal antibody detects all three isoforms of the SAPK1/JNK proteins only when they are activated by dual phosphorylation at threonine₁₈₃/tyrosine₁₈₅. Binding of polyclonal phospho-JNK antibody (at a dilution of 1:20) from NEB to TAMRA-labelled P1°- and P1*° peptides (each 5 nM) was measured by fluorescence polarization.
Affinity of polyclonal phospho-JNK antibody for peptide P1*°-TAMRA: K_{D} = 2.6 nM

The results of this experiment are illustrated in Fig. 2.

### Example 2:

### Determination of IC50 for Peptide P1°:

ompetition of NEB antibody-P1*°-TAMRA complex with P1*° eptide was measured by fluorescence correlation spectroscopy: inding of polyclonal phospho-SAPK1/JNK antibody from NEB (1:20 iluted) to TAMRA-labelled peptide P1*° (5 nM) was competed with non-fluorescent, double-phosphorylated peptide P1*°. Control peptides: mono-phosphorylated P1° and P1*.
Peptide P1*°: IC₅₀ = 5.0 nM ± 3.3 nM

The results of this experiment are illustrated in Fig. 3.

### Example 3:

### Determination of IC50 for Peptide P1° (under MKK7 enzyme assay conditions).

Competition of NEB antibody-P1*°-TAMRA complex with P1*° in presence of P1° substrate peptide (reflecting MKK7 assay conditions) was measured by fluorescence polarization: Binding of polyclonal phospho-JNK antibody from NEB #9251 (at a dilution of 1:20) to TAMRA-labelled peptide P1*° (5 nM), competition with P1*° peptide (0.1, 0.5, 2, 5, 20, 50, 200, 2000 nM) in Hepes buffer, MgCl₂ 10 mM, ATP 20 µM, Pluronic 0.1%, P1° substrate peptide 100 µM.
Peptide P1*°: IC₅₀ = 35.1 nM ± 10.6 nM

The results of this experiment are illustrated in Fig. 4.

### Example 4:

### Determination of Km for peptide P1°(Read-out: fluorescence polarization):

Assay conditions: MKK7 kinase (80 nM), P1° peptide (0, 10, 30, 70, 100, 200, 500 µM), ATP (100 µM), NEB antibody #9251 (1:20) diluted and P1*° peptide-TAMRA (5 nM) at RT (room temperature).
Kₘ = 94 nM ± 59 nM, Vₘₐₓ = 94.6 nM/min ± 0.03 nM/min

The results of this experiment are illustrated in Fig. 5.

### Example 5:

### Determination of Km for ATP (Read-out: fluorescence polarization) :

Assay conditions: MKK7 (80 nM), P1° peptide (100 µM), ATP (0, 1, 5, 10, 20, 50, 100, 200, 500 µM), incubation time to 2h at RT, polyclonal active-JNK antibody from NEB #9251 (1:20 dilution) and P1*° peptide-TAMRA at (5 nM).
Kₘ = 17.3 nM ± 18.1 nM, Vₘₐₓ = 0.28 mP/min ± 0.07 mP/min

The results of this experiment are illustrated in Fig. 6.

### Example 6:

### Inhibition of MKK7 activity with Staurosporine (Read-out: fluorescence polarization):

Assay conditions: MKK7 (0.3µM), P1° peptide (100 µM), ATP (10 µM), Staurosporine (0, 0.05, 0.2, 1, 2, 5, 10 µM), polyclonal active-JNK antibody from NEB #9251 (1:20 dilution) and P1*° peptide-TAMRA at (5 nM).
IC₅₀ = 63.3 nM ± 10.2 nM

The results of this experiment are illustrated in Fig. 7.

### Example 7:

### Fluorescence Polarization measurements of MKK7 activity (time course) :

In this experiment, the time course has been investigated at P1° substrate concentrations of 100 µM. The reactions were stopped with EDTA.

The following reagents have been used (the compositions of the buffers and specifications of the reagents are explained in more detail later on):
Hepes buffer, MgCl₂ 10 mM, ATP 20 µM, P1° peptide at 100 µM, NEB antibody # 9251 1:20 dilution, P1*°-TAMRA 5 nM, Pluronic 0.1%, MKK7 at 0, 0.005, 0.02, 0.08, 0.2, 0.5 and 2 µM.
After addition of MKK7, the enzyme reactions were performed in Nunc chambers at RT. The samples were continuously measured at different time points by Fluorescence polarization.

| | | | | | **0 h** |
|---|---|---|---|---|---|
| **Conjugate: P1*°-Tamra** | | | | | 39,7 |
| **High: P1*°-Tamra + NEB ab (1:20)** | | | | | 139,1 |
| **Low: P1*°-Tamra + NEB ab (1:20) + P1*° 200 nM** | | | | | 84,5 |

| ***100 µM P1°*** | **0 h** | **0.5 h** | **1h** | **1.5 h** | **3 h** |
|---|---|---|---|---|---|
| **MKK7: 0.0 µM** | 134.7 | 139.1 | 139.7 | 140.2 | 144.8 |
| **MKK7: 0.005 µM** | 131.5 | 137.4 | 136.8 | 136.9 | 138.1 |
| **MKK7: 0.02 µM** | 135.4 | 140.3 | 140.9 | 139.7 | 140.0 |
| **MKK7: 0.08 µM** | 133.5 | 137.6 | 135.8 | 128.4 | 115.2 |
| **MKK7: 0.2 µM** | 135.3 | 132.1 | 121.9 | 113.8 | 104.7 |
| **MKK7: 0.5 µM** | 135.3 | 125.5 | 115.3 | 108.8 | 106.6 |
| **MKK7: 1.0 µM** | 135.7 | 19.6 | 112.2 | 105.3 | 108.4 |

| | | | | | |
|---|---|---|---|---|---|
| Fluorescence Polarization (FP) values are given in mP. | | | | | |

### Example 8:

### Fluorescence Polarization measurements of MKK7 activity (time course) :

In this experiment the assay conditions have been optimized. The reactions were stopped with EDTA.

Two different assay strategies were investigated: first, the NEB read-out antibody was included in the enzyme reaction (STOP solution: contains only EDTA and P1*°-TAMRA); second, the NEB read-out antibody was included in the STOP solution.

The following reagents have been used (explanations later on): Hepes buffer, MgCl₂ (10 mM), ATP (20 µM), P1° peptide (100 µM), polyclonal antibody NEB #9251 (1:20), P1*°-TAMRA (5 nM), Pluronic (0.1%), BSA (0.01%), MKK7 at (0.04, 0.08 and 0.2 µM), EDTA (10 mM) (all final conc.).

The enzyme reactions were performed in Eppendorf reaction tubes at RT. Aliquots of 20 µl were withdrawn from the reactions at different time points and mixed with 10 µl STOP solution. The stopped reactions were incubated at RT for at least 30 min and measured by Fluorescence Polarization.

### Results:

| | | | | | | **0 h** |
|---|---|---|---|---|---|---|
| **Conjugate: P1*°-Tamra** | | | | | | 33,2 |
| **High: P1*°-Tamra + NEB ab (1:20)** | | | | | | 145,7 |
| **Low: P1*°-Tamra + NEB ab (1:20) + P1*° 200 nM** | | | | | | 71,5 |

| ***NEB ab in reaction*** | **0 h** | **0.5 h** | **1 h** | **1.5 h** | **2 h** | **3 h** |
|---|---|---|---|---|---|---|
| **MKK7: 0.04 µM** | 150.0 | 128.5 | 125.7 | 112.9 | 109.7 | 78.6 |
| **MKK7: 0.08 µM** | 138.5 | 122.5 | 105.9 | 86.4 | 84.5 | 65.0 |
| **MKK7: 0.2 µM** | 137.2 | 101.4 | 83.1 | 78.1 | 72.1 | 59.4 |
| ***NEB ab in STOP sol.*** | | | | | | |
| **MKK7: 0.04 pM** | 147.2 | 141.5 | 140.2 | 135.8 | 134.2 | 138.2 |
| **MKK7: 0.08 µM** | 143.3 | 139.1 | 133.6 | 122.1 | 115.8 | 125.0 |
| **MKK7: 0.2 µM** | 148.2 | 130.2 | 114.0 | 102.8 | 99.2 | 119.4 |

| | | | | | | |
|---|---|---|---|---|---|---|
| Fluorescence Polarization (FP) values are given in mP. | | | | | | |

### Example 9:

### Fluorescence Polarization measurements of MKK7 activity (time course) in Nanocarrier plates (supplier: EVOTEC BioSystems AG, 1.2 µl volume):

### Assay conditions (end conc.):

1. P1° substrate 100 µM, polyclonal antibody NEB #9251 (1:20), ATP 20 µM, MgCl 10 mM
2. MKK7 enzyme: 0, 25, 50, 75, 100, 200, 350, 500 nM
3. STOP solution: EDTA 10 mM, P1*°-TAMRA (5 nM)

### Stock solutions:

1. P1° substrate 125 µM, NEB ab (1:13.3), ATP 25 µM, MgCl 12.5 mM
2. MKK7 enzyme: 2500 nM
3. STOP solution: EDTA 60 mM, P1-TAMRA 30 nM

Solutions 1. - 3. in Hepes buffer, Pluronic 0.1%, BSA 0.01%.

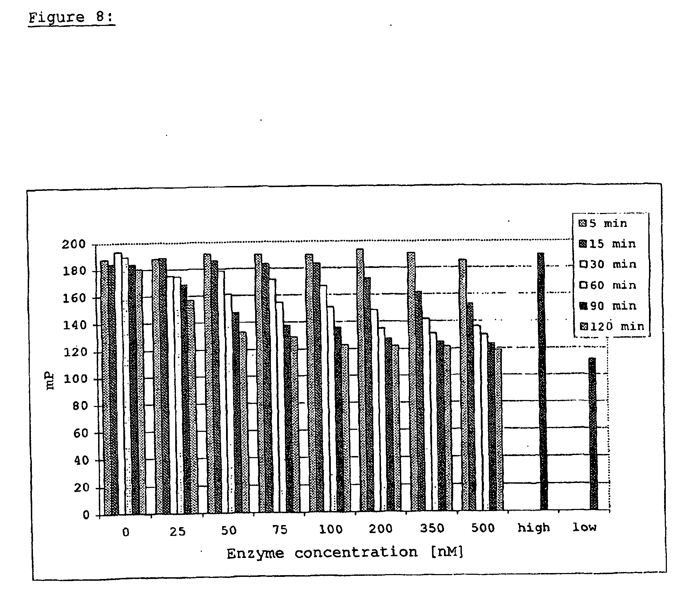

It has been revealed that bis-phosphorylated-TAMRA-P1*°-peptide (ligand) is bound by the polyclonal anti-active JNK-antibody, which is called "high" or "positive control". Bis-phosphory-lated peptide P1*° can be used as competitor, resulting in free labelled ligand called "low" or "negative control". This can be seen in Figure 8 where FP values are given in mP and the bars illustrate the time course at different enzyme concentrations.

### List of reagents of the MKK7 kinase assay:

### List of used JNK1-peptides:

- P1°: H-Lys-Phe-Met-Met-Thr-Pro-pTyr-Val-Val-Thr-Arg-NH2
- P1*: H-Lys-Phe-Met-Met-pThr-Pro-Tyr-Val-Val-Thr-Arg-NH2
- P1*°: H-Lys-Phe-Met-Met-pThr-Pro-pTyr-Val-Val-Thr-Arg-NH2
- TAMRA-P1*°:

5-TAMRA-AEEA-Lys-Phe-Met-Met-pThr-Pro-pTyr-Val-Val-Thr-Arg-NH2

### Ligand:

TAMRA-P1*°-Peptide: 5-TAMRA-AEEA-Lys-Phe-Met-Met-pThr-Pro-pTyr-Val-Val-Thr-Arg-NH2
Supplier: EVOTEC BioSystems AG, solid-phase peptide synthesis HK-03-65-P1-13; M = 2089 g/mol; MALDI (2092.18)

1mM stock solution in 100% DMSO
The stock should be aliquoted in 10 µl aliquots and kept at - 20°C.
5nM working solution.

### Competitor:

P1*°-Peptide: H-Lys-Phe-Met-Met-pThr-Pro-pTyr-Val-Val-Thr-Arg-NH2
Supplier: EVOTEC BioSystems AG, solid-phase peptide synthesis HK-03-60-P1-7; M = 1532 g/mol; MALDI (1531.88)

10mM stock solution in 100% DMSO
The stock should be aliquoted in 10 µl aliquots and kept at - 20°C.
200µM working solution.

### Substrate: MKK7 kinase substrate

P1°-Peptide: H-Lys-Phe-Met-Met-Thr-Pro-pTyr-Val-Val-Thr-Arg-NH2
Supplier: EVOTEC BioSystems AG, solid-phase peptide synthesis HK-03-58-HF; M = 1451 g/mol; MALDI (1453.57)

10mM stock solution in 100% DMSO
The stock should be aliquoted in 10 µl aliquots and kept at - 20°C.
100µM working solution.

### Enzyme:

MKK7 kinase Supplier: Upstate Biotech cat. no. 14-366, lot no. 19105 and no. 20658.
   See enzyme activity specifications on the respective certificate of analysis.
   Specific activity: Approximately 20 Units/mg when max. activated.
   20 µg of enzyme (9 µM) in 50µl of 50 mM Tris-HCl, pH 7.5, 150 mM NaCl, 270 mM sucrose, 0.1 mM EGTA, 0.1% 2-mercaptoethanol, 0.03% Brij-35, 1 mM benzamidine, 0.2 mM PMSF.
   The stock was kept at -70°C. Freezing and thawing cycles were avoided.

### Anti active SAPK1/JNK-specific antibody:

### New England Biolabs, U.S.A.: NEB #9251

Anti active JNK polyclonal antibody purified via affinity chromatography (Protein A) from rabbit serum, conc.: 0.02 mg/ml, equiv. 130 nM. Storage buffer: 10 mM Hepes pH 7.5, 150 mM NaCl, 100 µg/ml BSA, 50 % glycerol, store at -70 °C. The stock should be aliquoted in 10 µl aliquots and kept at -20°C. Working solution: 1:20 dilution in 1x Assay-Buffer/0.1% Pluronic

### Reagents and buffers:

*Assay buffer:*
*10x HEPES-Assay-Buffer pH 7.5*
*500mM HEPES*
*1mM EGTA*
*100mM DTT*
*62.5mM NaCl*

Dissolve 770mg DTT (FLUKA cat. no. 43815; MW 154.25g/mol) [final concentration 100mM] in 30 ml of Millipore water, add 625µl of 5M NaCl₂ [final concentration 62.5mM], 19.02mg EGTA (Merck cat. No.1.06404; MW 380.35g/mol) [final concentration 1mM] and 5.96g HEPES (FLUKA cat. no. 54457) [final concentration 500mM] and adjust pH with 1M NaOH to pH 7.5. Top up to 50 ml with Millipore water. The buffer was filtered sterile (0.22µm) aliquoted to 1.5ml and was stored at -20°C.

### 1M MgCl₂:

Dissolve 10.2 g MgCl₂.6H₂O (FLUKA cat. no. 63068) in 50 ml of Millipore water. Buffer was filtered (0.42µm) and can be stored at 4°C for several months.

### 1% (w/v) Pluronic F-127

Dissolve 0.5 g Pluronic (Sigma, cat. no. P-2443) in 50 ml of Millipore water. Stir gently to get a clear solution. Solution was filtered (0.42µm) and can be stored at 4°C for several months.

### 1x REPES-Assay-Buffer/0.1% Pluxonic pH 7.5: (15ml) :

*50mM HEPES*
*0.1mM EGTA*
*10mM DTT*
*0.1% Pluronic*

Add 1.5 ml 1% (w/v) Pluronic F-127 in water and 1.5 ml of 10x HEPES-Assay-Buffer pH 7.5.to 12 ml of Millipore water. Stir the mixture and check the pH value. The mixture could be stored at 4°C for 1-2 weeks.

### 0.5M EDTA (50ml) :

Dissolve 9.309g EDTA (Roche Molecular Biochemicals, cat. no. 808288) in 40 ml of Millipore water and adjust pH to 8-9 with 10M NaOH in order to get a clear solution. Top up to 50 ml with Millipore water. Buffer was filtered (0.42µm) and can be stored at 4°C for several months.

### 10mM ATP (15ml):

Dissolve 9.1mg ATP (Roche Molecular Biochemicals, cat. no. 126888) in 1.5 ml 1x HEPES-Assay-Buffer/0.1% Pluronic pH 7.5. The solution was filtered (0.42µm) and can be stored at -20°C for 2 weeks.

### DMSO: 100% DMSO

The solvent was purchased from SIGMA (cat. No. P-2650), sterile filtered.

## Claims

1. A process for detecting threonine or serine kinase activity in an immunoassay comprising the following steps:
a) providing a protein or peptide comprising the sequence motif
-Z-X-Y- or -Y-X-Z-
wherein
Z = threonine or serine
X = a sequence of amino acids between 1 and 1000 amino acids which may be the same or different
Y = tyrosine, threonine or serine
as a substrate for threonine or serine kinase, said protein or peptide being pre-phosphorylated at the Y position;
b) incubating the protein or peptide with a phosphate donor and a threonine or serine kinase to form a protein or peptide which is phosphorylated at positions Y and Z;
c) adding an antibody having a specificity to a peptide or protein which is phosphorylated at the Y and Z position; and
d) detecting the threonine or serine kinase activity.

2. The process according to claim 1 wherein the phosphate donor is ATP, GTP, or a synthetic cosubstrate.

3. The process according to claim 1 or 2 wherein the immunoassay is performed as a direct binding immunoassay, preferably a homogeneous direct binding immunoassay.

4. The process according to claim 3 wherein a labelled peptide or protein is used as a substrate.

5. The process according to claim 3 wherein a labelled antibody is used.

6. The process according to claim 4 or 5 wherein the peptide/protein or antibody is labelled by a luminescent tag, a radioactive marker, a reporter enzyme or an affinity ligand.

7. The process according to claim 1 or 2 wherein the immunoassay is performed as an indirect binding immunoassay, preferably a homogeneous indirect binding immunoassay.

8. The process according to claim 7 wherein a labelled ligand which is phosphorylated at its Y and Z position (bis-phosphorylated ligand) is added to compete with the protein or peptide which is phosphorylated at its Y and Z position (bis-phosphorylated protein or peptide) for binding to the antibody.

9. The process according to claim 8 wherein the bis-phosphorylated ligand is labelled by a luminescent tag, a radioactive marker, a reporter enzyme or an affinity ligand.

10. The process according to claim 9 wherein the ligand comprises Lys-Phe-Met-Met-pThr-Pro-pTyr-Val-Val-Thr-Arg-NH₂.

11. The process according to at least one of claims 1 to 10 wherein the assay is performed as a fluorescence immunoassay, in particular a fluorescence polarization immunoassay, a fluorescence correlation spectroscopic assay, a fluorescence resonance energy transfer assay, or a fluorescence intensity distribution assay.

12. The process according to at least one of claims 1 to 11 wherein X in the sequence motif comprises proline or glutamate or glycine.

13. The process according to at least one of claims 1 to 12 wherein the protein provided is JNK1, JNK2 or JNK3 protein.

14. The process according to at least one of claims 1 to 12 wherein the peptide provided includes sequences identical to those of the JNK1, JNK2 or JNK3 active-site loop.

15. The process according to claims 1 to 12 wherein the peptide comprises the amino acid sequence H-Lys-Phe-Met-Met-Thr-Pro-pTyr-Val-Val-Thr-Arg-NH₂ (p means phosphory-lated).

16. The process according to claims 1 to 12 wherein the peptide is composed of the amino acid sequence H-Lys-Phe-Met-Met-Thr-Pro-pTyr-Val-Val-Thr-Arg-NH₂ (p means phosphorylated).

17. The process according to at least one of claims 1 to 16 wherein the incubation of the protein or peptide is carried out in the prescence of a threonine kinase.

18. The process according to claim 17 wherein the threonine kinase is a mitogen-activated protein kinase kinase (MKK) (also named stress activated protein kinase kinase, SKK).

19. The process according to claim 18 wherein the kinase is MKK7 (also named SKK4).

20. The process according to at least one of claims 1 to 19 wherein the antibody is a monoclonal or polyclonal antibody.

21. The process according to claim 20 wherein the antibody is a polyclonal antibody.

22. The process according to claim 21 wherein the antibody is a polyclonal antibody specific for bis-phosphorylated, in particular active JNK.

23. The process according to at least one of claims 1 to 22 wherein steps a) to d) are performed sequentially.

24. A kit for detecting threonine or serine kinase activity in an immunoassay comprising the following components:
- a substrate as defined in claim 1;
- an antibody as defined in claim 1.

25. The kit according to claim 24 further comprising a threonine or serine kinase, and/or reaction buffers including a phosphate donor, preferably ATP.

26. The kit according to claim 24 or 25 further comprising a labelled ligand, preferably luminescently labelled ligand, said ligand comprising the following sequence motif
-Z-X-Y- or -Y-X-Z-
wherein
Z = threonine or serine
X = a sequence of amino acids between 1 and 1000 amino acids which may be the same or different
Y = tyrosine, threonine or serine
said protein or peptide ligand being phosphorylated at the Z and Y positions.

27. A labelled ligand for use in a serine/threonine kinase assay comprising the sequence motif according to claim 26.

28. A use of the assay process according to at least one of the claims 1 to 23 or the kit according to claims 24 to 26 or the ligand according to claim 27 for screening modulators for threonine or serine kinase activity, in particular inhibitors for a threonine or serine kinase, or for detecting novel threonine or serine kinases.

## Patentansprüche

1. Verfahren zum Nachweis von Threonin- oder Serinkinase-Aktivität in einem Immunoassay, umfassend die folgenden Schritte:
a) Bereitstellung eines Proteins oder Peptids, umfassend das Sequenzmotiv
-Z-X-Y- oder -Y-X-Z-
worin
Z = Threonin oder Serin
X = eine Sequenz von Aminosäuren zwischen 1 und 1000 Aminosäuren, welche gleich oder verschieden sein können,
Y = Tyrosin, Threonin oder Serin
als Substrat für Threonin- oder Serinkinase, wobei das Protein oder Peptid an der Y-Position vorphosphoryliert ist;
b) Inkubation des Proteins oder Peptids mit einem Phosphatdonor und einer Threonin- oder Serinkinase, um ein Protein oder Peptid zu bilden, welches an den Positionen Y und Z phosphoryliert ist;
c) Zugabe eines Antikörpers, der eine Spezifität für ein Peptid oder Protein aufweist, das an der Y- und Z-Position phosphoryliert ist; und
d) Nachweis der Threonin- oder Serinkinase-Aktivität.

2. Verfahren nach Anspruch 1, wobei der Phosphatdonor ATP, GTP oder ein synthetisches Kosubstrat ist.

3. Verfahren nach Anspruch 1 oder 2, wobei der Immunoassay als ein Immunoassay mit direkter Bindung, vorzugsweise ein homogener Immunoassay mit direkter Bindung, durchgeführt wird.

4. Verfahren nach Anspruch 3, wobei ein markiertes Peptid oder Protein als Substrat verwendet wird.

5. Verfahren nach Anspruch 3, wobei ein markierter Antikörper verwendet wird.

6. Verfahren nach Anspruch 4 oder 5, wobei das Peptid/Protein oder der Antikörper mit einer Lumineszenzmarkierung, einem radioaktiven Marker, einem Reporterenzym oder einem Affinitätsliganden markiert ist.

7. Verfahren nach Anspruch 1 oder 2, wobei der Immunoassay als ein Immunoassay mit indirekter Bindung, vorzugsweise ein homogener Immunoassay mit indirekter Bindung, durchgeführt wird.

8. Verfahren nach Anspruch 7, wobei ein markierter Ligand, der an seiner Y- und Z-Position phosphoryliert ist, (bisphosphorylierter Ligand) zugegeben wird, um mit dem Protein oder Peptid, welches an seiner Y- und Z-Position phosphoryliert ist, (bisphosphoryliertes Protein oder Peptid) um die Bindung an den Antikörper zu konkurrieren.

9. Verfahren nach Anspruch 8, wobei der bisphosphorylierte Ligand mit einer Lumineszenzmarkierung, einem radioaktiven Marker, einem Reporterenzym oder einem Affinitätsliganden markiert ist.

10. Verfahren nach Anspruch 9, wobei der Ligand Lys-Phe-Met-Met-pThr-Pro-pTyr-Val-Val-Thr-Arg-NH₂ umfasst.

11. Verfahren nach mindestens einem der Ansprüche 1 bis 10, wobei der Assay als ein Fluoreszenzimmunoassay, insbesondere ein Fluoreszenzpolarisationimmunoassay, ein Fluoreszenzkorrelationsspektroskopie-Assay, ein Fluoreszenzresonanzenergietransfer-Assay oder ein Fluoreszenzintensitätsverteilungs-Assay, durchgeführt wird.

12. Verfahren nach mindestens einem der Ansprüche 1 bis 11, wobei X in dem Sequenzmotiv Prolin oder Glutamat oder Glycin umfasst.

13. Verfahren nach mindestens einem der Ansprüche 1 bis 12, wobei das bereitgestellte Protein ein JNK1-, JNK2- oder JNK3-Protein ist.

14. Verfahren nach mindestens einem der Ansprüche 1 bis 12, wobei das bereitgestellte Peptid Sequenzen einschließt, welche mit denjenigen der Schleife der aktiven Stelle von JNK1, JNK2 oder JNK3 identisch sind.

15. Verfahren nach den Ansprüchen 1 bis 12, wobei das Peptid die Aminosäuresequenz H-Lys-Phe-Met-Met-Thr-Pro-pTyr-Val-Val-Thr-Arg-NH₂ (p bedeutet phosphoryliert) umfasst.

16. Verfahren nach den Ansprüchen 1 bis 12, wobei das Peptid aus der Aminosäuresequenz H-Lys-Phe-Met-Met-Thr-Pro-pTyr-Val-Val-Thr-Arg-NH₂ (p bedeutet phosphoryliert) besteht.

17. Verfahren nach mindestens einem der Ansprüche 1 bis 16, wobei die Inkubation des Proteins oder Peptids in Gegenwart einer Threoninkinase durchgeführt wird.

18. Verfahren nach Anspruch 17, wobei die Threoninkinase eine mitogenaktivierte Proteinkinasekinase (MKK) (auch als stressaktivierte Proteinkinasekinase, SKK, bezeichnet) ist.

19. Verfahren nach Anspruch 18, wobei die Kinase MKK7 (auch als SKK4 bezeichnet) ist.

20. Verfahren nach mindestens einem der Ansprüche 1 bis 19, wobei der Antikörper ein monoklonaler oder polyklonaler Antikörper ist.

21. Verfahren nach Anspruch 20, wobei der Antikörper ein polyklonaler Antikörper ist.

22. Verfahren nach Anspruch 21, wobei der Antikörper ein polyklonaler Antikörper ist, welcher für bisphosphoryliertes, insbesondere aktives JNK spezifisch ist.

23. Verfahren nach mindestens einem der Ansprüche 1 bis 22, wobei die Schritte a) bis d) sequenziell durchgeführt werden.

24. Kit zum Nachweis von Threonin- oder Serinkinase-Aktivität in einem Immunoassay, umfassend die folgenden Komponenten:
- ein Substrat wie in Anspruch 1 definiert;
- einen Antikörper wie in Anspruch 1 definiert.

25. Kit nach Anspruch 24, ferner umfassend eine Threonin-oder Serinkinase und/oder Reaktionspuffer, die einen Phosphatdonor, vorzugsweise ATP, einschließen.

26. Kit nach Anspruch 24 oder 25, ferner umfassend einen markierten Liganden, vorzugsweise einen lumineszenzmarkierten Liganden, wobei der Ligand das folgende Sequenzmotiv
-Z-X-Y- oder -Y-X-Z-
Z = Threonin oder Serin
X = eine Sequenz von Aminosäuren zwischen 1 und 1000 Aminosäuren, welche gleich oder verschieden sein können,
Y = Tyrosin, Threonin oder Serin,
wobei der Protein- oder Peptid-Ligand an den Z- und Y-Positionen phosphoryliert ist.

27. Markierter Ligand zur Verwendung in einem Serinkinase/Threoninkinase-Assay, umfassend das Sequenzmotiv nach Anspruch 26.

28. Verwendung des Assay-Verfahrens nach mindestens einem der Ansprüche 1 bis 23 oder des Kits nach den Ansprüchen 24 bis 26 oder des Liganden nach Anspruch 27 zum Screenen hinsichtlich von Modulatoren von Threonin- oder Serinkinase-Aktivität, insbesondere Inhibitoren für eine Threonin- oder Serinkinase, oder zum Nachweis neuer Threonin- oder Serinkinasen.

## Revendications

1. Procédé pour détecter l'activité de thréonine ou sérine kinase dans un immunodosage, comprenant les étapes suivantes :
a) mise à disposition d'une protéine ou d'un peptide, comprenant le motif de séquence :
-Z-X-Y- ou -Y-X-Z-
où
Z = thréonine ou sérine,
X = une séquence des acides aminés, avec 1 à 1000 acides aminés, qui peuvent être identiques ou différents,
Y = tyrosine, thréonine ou sérine,
comme substrat d'une thréonine ou sérine kinase, ladite protéine ou peptide étant préphosphorylé en la position Y ;
b) incubation de la protéine ou du peptide avec un donneur de phosphate et une thréonine ou sérine kinase, pour former une protéine ou un peptide qui est phosphorylé en les positions Y et Z ;
c) addition d'un anticorps ayant une spécificité pour un peptide ou une protéine, qui est phosphorylé en les positions Y et Z, et
d) détection de l'activité de thréonine ou sérine kinase.

2. Procédé selon la revendication 1, dans lequel le donneur de phosphate est l'ATP, le GTP ou un co-substrat synthétique.

3. Procédé selon la revendication 1 ou 2, dans lequel l'immunodosage est réalisé en tant qu'immunodosage à liaison directe, de préférence un immunodosage homogène à liaison directe.

4. Procédé selon la revendication 3, dans lequel on utilise comme substrat, un peptide ou une protéine marquée.

5. Procédé selon la revendication 3, dans lequel on utilise un anticorps marqué.

6. Procédé selon la revendication 4 ou 5, dans lequel le peptide/protéine ou l'anticorps est marqué avec un marqueur luminescent, un marqueur radioactif, une enzyme reporter ou un ligand d'affinité.

7. Procédé selon la revendication 1 ou 2, dans lequel l'immunodosage est réalisé comme un immunodosage à liaison indirecte, de préférence un immunodosage homogène à liaison indirecte.

8. Procédé selon la revendication 7, dans lequel un ligand marqué, qui est phosphorylé en ses positions Y et Z (ligand bis-phosphorylé) est ajouté pour entrer en compétition avec la protéine ou le peptide, qui est phosphorylé en ses positions Y et Z (protéine ou peptide bis-phosphorylé), pour la liaison à l'anticorps.

9. Procédé selon la revendication 8, dans lequel le ligand bis-phosphorylé est marqué avec un marqueur luminescent, un marqueur radioactif, une enzyme reporter ou un ligand d'affinité.

10. Procédé selon la revendication 9, dans lequel le ligand comprend Lys-Phe-Met-pThr-Pro-pTyr-Val-Val-Thr-Arg-NH₂.

11. Procédé selon au moins l'une des revendications 1 à 10, dans lequel le dosage est réalisé comme un immunodosage à fluorescence, en particulier un immunodosage à polarisation de fluorescence, un dosage spectroscopique à corrélation de fluorescence, un dosage de transfert d'énergie de résonance de fluorescence, ou un dosage de distribution d'intensité de fluorescence.

12. Procédé selon au moins l'une des revendications 1 à 11, dans lequel X dans le motif de séquence comprend une proline ou un glutamate ou une glycine.

13. Procédé selon au moins l'une des revendications 1 à 12, dans lequel la protéine procurée est la protéine JNK1, JNK2 ou JNK3.

14. Procédé selon au moins l'une des revendications 1 à 12, dans lequel le peptide procuré comprend des séquences identiques à celles de la boucle de site actif de JNK1, JNK2 ou JNK3.

15. Procédé selon les revendications 1 à 12, dans lequel le peptide comprend la séquence des acides aminés H-Lys-Phe-Met-Met-Thr-Pro-pTyr-Val-Val-Thr-Arg-NH₂ (p signifie phosphorylé).

16. Procédé selon les revendications 1 à 12, dans lequel le peptide est composé de la séquence des acides aminés H-Lys-Phe-Met-Met-Thr-Pro-pTyr-Val-Val-Thr-Arg-NH₂ (p signifie phosphorylé).

17. Procédé selon au moins l'une des revendications 1 à 16, dans lequel l'incubation de la protéine ou du peptide est réalisée en la présence d'une thréonine kinase.

18. Procédé selon la revendication 17, dans lequel la thréonine kinase est une protéine kinase kinase activée par le mitogène (MKK) (également dite protéine kinase kinase activée par un stress, SKK).

19. Procédé selon la revendication 18, dans lequel la kinase est la MKK7 (également dite SKK4).

20. Procédé selon au moins l'une des revendications 1 à 19, dans lequel l'anticorps est un anticorps monoclonal ou polyclonal.

21. Procédé selon la' revendication 20, dans lequel l'anticorps est un anticorps polyclonal.

22. Procédé selon la revendication 21, dans lequel l'anticorps est un anticorps polyclonal, spécifique d'un JNK bis-phosphorylé, en particulier actif.

23. Procédé selon au moins l'une des revendications 1 à 22, dans lequel les étapes a) à d) sont réalisées séquentiellement.

24. Kit de détection de l'activité de thréonine ou sérine kinase dans un immunodosage, comprenant les composants suivants :
- un substrat tel que défini à la revendication 1,
- un anticorps tel que défini à la revendication 1.

25. Kit selon la revendication 24, comprenant en outre une thréonine ou sérine kinase, et/ou des tampons de réaction comprenant un donneur de phosphate, de préférence l'ATP.

26. Kit selon la revendication 24 ou 25, comprenant en outre un ligand marqué, de préférence un ligand marqué de manière luminescente, ledit ligand comprenant le motif de séquence :
-Z-X-Y- ou -Y-X-Z-
où
Z = thréonine ou sérine,
X = une séquence des acides aminés, avec 1 à 1000 acides aminés, qui peuvent être identiques ou différents,
Y = tyrosine, thréonine ou sérine,
ledit ligand protéine ou peptide étant phosphorylé en les positions Z et Y.

27. Ligand marqué à utiliser dans un dosage de sérine/thréonine kinase, comprenant le motif de séquence selon la revendication 26.

28. Utilisation du processus de dosage selon au moins l'une des revendications 1 à 23 ou du kit selon les revendications 24 à 26 ou du ligand selon la revendication 27, pour cribler des modulateurs pour l'activité de thréonine ou sérine kinase, ou pour détecter de nouvelles thréonine ou sérine kinases.
